# EUROPÄISCHE PATENTSCHRIFT

(11) **EP 0 615 409 B1**
(45) Veröffentlichungstag und Bekanntmachung des Hinweises auf die Patenterteilung: **05.03.1997**
(21) Anmeldenummer: 92922163.8
(22) Anmeldetag: 04.11.1992
(51) Int. Cl.: A01N 63/04, C12N 1/14

(54) **STÄMME DES PILZES TRICHODERMA, DARAUS HERGESTELLTES FUNGIZID, SOWIE VERFAHREN ZU DESSEN ANWENDUNG**
STRAINS OF THE TRICHODERMA FUNGUS, FUNGICIDE DERIVED THEREFROM AND PROCESS FOR USING THE SAME
SOUCHES DU CHAMPIGNON TRICHODERMA, FONGICIDE EXTRAIT DE CELLES-CI ET SON PROCEDE D'UTILISATION

(30) Priorität: 05.11.1991 AT 2191/91
(43) Veröffentlichungstag der Anmeldung: 21.09.1994
(73) Patentinhaber: LIGNOCELL HOLZ-BIOTECHNOLOGIE GmbH, A-8607 Kapfenberg (AT)
(72) Erfinder: MESSNER, Kurt, A-1170 Wien (AT); FLECK, Vinzenz, A-8130 Frohnleiten (AT); BUERGEL, Jochen, A-1160 Wien (AT)
(86) Internationale Anmeldenummer: AT9200138
(87) Internationale Veröffentlichungsnummer: WO9308694

(56) Entgegenhaltungen:
- EP-A- 0 383 201
- GB-A- 2 239 800

## Beschreibung

Gegenstand der Erfindung ist jeweils ein Stamm der Pilze Trichoderma harzianum LC 1-3, ein Fungizid, das sie enthält und als Holzschutzmittel oder Pflanzenschuzmittel eingesetzt werden kann, sowie ein Verfahren zur Bekämpfung von Pilzen, welche Holz, lebende Bäume oder Pflanzen schädigen.

Holz wird, wenn es mehr als 20 % Feuchte enthält, von Pilzen befallen und geschädigt. Schutz vor Pilzbefall ist in der Gefährdungsklasse 2 (bei möglicher vorübergehender Befeuchtung), vor allem aber in den Gefährdungsklassen 3 (der Witterung und Kondensation ausgesetzt, aber nicht im Erdkontakt) und 4 (im dauernden Erdkontakt) erforderlich. Holzschäden können in Form von Fäulnis (Braun-, Weiß-, Moderfäule) oder als Verfärbung (Bläue) auftreten. Die intensivste Schädigung erfolgt durch die Braunfäulepilze (z.B Hausschwamm).

Die Verwendung einiger der wirksamsten chemischen Holzschutzmittel wird derzeit von den Behörden als bedenklich betrachtet und in manchen Ländern verboten. Grund dafür ist ihre geringe Umweltverträglichkeit bei Auswaschung, vor allem aber bei der Entsorgung des Holzes. Ebenso wird der Einsatz einiger Pflanzenschutzmittel wegen ihrer schlechten Abbaubarkeit im Boden und der damit verbundenen Anreicherung in Frage gestellt.

Vertreter der Gattung Trichoderma kommen zahlreich im Boden vor und sind als natürliche Antagonisten verschiedener Pilzarten bekannt. Der Vorteil eines auf der Verwendung von Trichoderma beruhenden Fungizids besteht darin, daß die von Trichoderma gebildeten Hemmstoffe biogenen Ursprungs sind und daher bei Auswaschung in den Boden oder bei der Entsorgung von Altholz keine ökologischen Risiken bestehen. Sie sind im Boden biologisch abbaubar. Die chemische Natur der Hemmstoffe läßt den Schluß zu, daß bei Verbrennung keine toxischen Emissionen entstehen.

Von keinem der bisher in der Literatur beschriebenen oder patentierten und in Holzschutzmitteln verwendeten Stämme einer Art von Trichoderma wurde nachgewiesen, daß er imstande ist, alle in der Europäischen Norm EN 113 "Bestimmung der Grenze der Wirksamkeit gegenüber holzzerstörenden Basidiomyceten, die auf Agar wachsen" angeführten Teststämme vollständig zu hemmen. Weiters ist die Hemmwirkung dieser Stämme meist auf spezifische Pilze oder Pilzgruppen beschränkt (DE-OS 3904710, DE-OS 3600394). Der toxikologische Status dieser Pilze wurde außerdem nie definiert.

Ziel der Erfindung war es, wirksamere Stämme von Trichoderma harzianum zu selektionieren, die außerdem gegen ein breites Spektrum von Schadpilzen anwendbar sind. Weiteres Ziel der Erfindung war es, Stämme zu selektionieren, die keine akute Toxizität aufweisen. Dieses Ziel wird erfindungsgemäß mit den Stämmen Trichoderma harzianum LC 1, Trichoderma harzianum LC 2 und Trichoderma harzianum LC 3 erreicht (Beispiel 1). Sie wurden nach einer Kombination von Methoden, welche dem Stand der Technik entsprechen, selektioniert. Die Stämme Trichoderma harzianum LC 2 und Trichoderma harzianum LC 3 sind Biovarianten des Stammes Trichoderma harzianum LC 1.

Die Stämme Trichoderma harzianum LC 1, LC 2 und LC 3 wurden vom Anmelder beim Centraalbureau voor Schimmelcultures, Oosterstraat 1, 3742 SK Baarn, Holland, gemäß dem Budapester Vertrag unter den Nummern CBS 595.91 (Trichoderma harzianum LC 1), CBS 596.91 (Trichoderma harzianum LC 2), CBS 597.91 (Trichoderma harzianum LC 3) hinterlegt. Hinterlegungsdatum ist der 4. November 1991.

Die hinterlegten Stämme haben sich in normgerechten Tests als nicht akut toxisch erwiesen (Beispiel 2). Vertreter der Gattung Trichoderma werden vom NIH als nicht pathogen für Mensch und Tier gewertet.

Die hinterlegten Stämme üben im Normtest EN 113 eine vollständige Hemmung auf die Testpilze aus (Beispiel 3). Darüber hinaus hemmen die beiden Stämme andere, phytopathologische Pilze wie Heterobasidion annosum und Armillaria mellea, welche lebende Bäume über die Wurzeln befallen, Obstbaumschädlinge wie z.B. Libertella blepharis, ebenso weitere pflanzenpathogene Pilze wie z.B. Ophiostoma ulmi, Botrytis cinerea, Pythium ultimum, Phytophthora Arten und Fusarium Arten (Beispiel 1).

Aus der großen Anzahl gehemmter Schadpilze geht hervor, daß die Stämme Trichoderma harzianum LC 1, LC 2 und LC 3 aufgrund ihrer starken Hemmwirkung gegen ein breites Spektrum von Pilzen und in vielen Anwendungsbereichen einsetzbar sind. Die Produktion eines einzigen Pilzstammes für mehrere Anwendungsbereiche führt, im Vergleich mit den bisher beschriebenen, nur spezifisch auf einzelne Pilze oder Pilzgruppen wirkenden Vertretern von Trichoderma, zu einem wesentlichen wirtschaftlichen Vorteil.

Das Fungizid enthält erfindungsgemäß koloniebildende Einheiten eines oder einer Kombination der Pilze Trichoderma harzianum LC 1-3 in für die Hemmung ausreichender Konzentration. Als koloniebildende Einheiten werden vorzugsweise Sporen in Suspension mit einer Keimzahl von 10⁴-10⁸ koloniebildenden Einheiten pro Milliliter eingesetzt. Mycelstücke können aber ebenfalls als koloniebildende Einheiten eingesetzt werden.

Mischungen des erfindungsgemäßen Fungizids mit anderen fungiziden oder nicht fungiziden Wirkstoffen wie z.B. Nährmedien, Puffer, Haftsubstanzen, Farben, UV-Schutzmitteln, Insektiziden, Akariziden, Herbiziden, Bakteriziden, Düngemitteln, Wachstumsregulatoren sind gegebenenfalls möglich. Die Kombination des erfindungsgemäßen Fungizids mit anderen Fungiziden in einer das Wachstum der erfindungsgemäßen Trichoderma Stämme nicht hemmenden Konzentration, ist zur Erzielung einer synergistischen Wirkung prinzipiell möglich. Erfindungsgemäß können der fungizide Pilz und die Zusatzstoffe auch getrennt voneinander, in mehreren Schritten appliziert werden.

Die möglichen Darreichungsformen des erfindungsgemäßen Fungizids können sein: Suspension, versprühbare Suspension, versprühbare Emulsion, Spritzpulver, emulgierbares Konzentrat, Granulat oder Mikrogranulat, Paste, Gel oder an Trägern wie Holz Agar- oder Alginat-Pellets immobilisiert.

Enthält ein Fungizid erfindungsgemäß koloniebildende Einheiten des Pilzes Trichoderma harzianum LC 3 in hemmender Konzentration, so kann aufgrund der nicht grünen, sondern weißen Sporen von Trichoderma harzianum LC 3 im Falle der Neubildung von Sporen an der Oberfläche von Holz keine grüne Verfärbung eintreten (Beispiel 4). Dies ist speziell bei Behandlung von Bläue auf Schnittholz, welches später weiterverarbeitet wird, von Bedeutung.

Das Verfahren zur Bekämpfung von Pilzen in Holz ist dadurch gekennzeichnet, daß das erfindungsgemäße Fungizid durch Tauchen, Streichen oder Sprühen appliziert wird. Die Applikation kann auch in Kombination mit anderen, zur Einbringung von Holzschutzmitteln gängigen Verfahren, wie z.B. dem Impfstichverfahren, erfolgen. Bei Applikation des Fungizids auf Holz ist eine anschließende Bebrütung des behandelten Holzes bei Optimaltemperatur und hoher Luftfeuchte, in einem Bebrütungsraum, ein weiterer Teil des erfindungsgemäßen Verfahrens, um die Keimung der Sporen und das Einwachsen der Pilzhyphen in das Holz zu fördern (Beispiel 5).

Die Applikation des erfindungsgemäßen Fungizids kann weiters in einer der Darreichungsformen gegen Schadpilze in lebenden Bäumen erfolgen, an Stellen, die im Zuge einer holzpathologischen Diagnose ermittelt wurden, Löcher gebohrt werden, durch die das Fungizid in das Stamminnere eingeführt wird (Beispiel 6).

Das erfindungsgemäße Fungizid kann weiters an den Wurzeln von Bäumen oder im Wurzelraum von Bäumen jeglichen Alters, zu deren Sanierung appliziert werden (Beispiel 7).

Weiters können zur Bekämpfung von Schadpilzen die Samen von Pflanzen mit dem erfindungsgemäßen Fungizid behandelt werden, oder dieses in hemmender Konzentration in die Erde eingebracht werden. Auf Stengeln oder Blättern wachsende Schadpilze wie Botrytis cinerea können durch Sprühen mit dem erfindungsgemäßen Fungizid bekämpft werden (Beispiel 8).

### Biologische Beispiele

### Herstellung der Sporensuspensionen:

2%iger Malzagar, wurde zu 15 ml in sterile Petrischalen abgefüllt und mit Sporen oder Mycel der Pilze Trichoderma LC 1, LC 2 bzw LC 3 beimpft. Nach 2 Wochen Bebrütung bei 25°C wurden die Sporen gewonnen, suspendiert und auf eine Heimzahl von 10⁸ Sporen/ml verdünnt. Damit wurden sterilisierte, gequollene Gerstenkörner in 1 l Erlenmeyerkolben beimpft. Nach 2 Wochen wurden die neu auf den Getreidekörnern gebildeten Sporen suspendiert und Suspensionen mit einer Keimzahl von 10⁴, 10⁶ bzw 10⁸ Sporen/ml hergestell.

### Beispiel 1:

### Laborprüfung auf gesteigerte Hemmwirkung der Trichoderma Stämme:

### a) Untersuchung auf flüchtige Hemmstoffe im Doppelplattentest:

Testpilz: Sterile Petrischalen wurden mit je 15 ml Malzagar gefüllt und in der Mitte des Agars ein, von einer lebenden Kultur von Lentinus lepideus, ein in Telegraphenmasten häufig vorkommender Schädling, ausgestochenes Agarplättchen mit 0.5 cm Durchmesser aufgelegt.
Trichoderma: In sterile Petrischalen wurden je 15 ml feuchtes Holzmehl eingefüllt und eben gestrichen. Dieses wurde mit je 1 ml Sporensuspension der zu untersuchenden Stämme von Trichoderma beimpft.
Testanordnung: Über den unteren Teil der Trichoderma Petrischalen wurde eine gasdurchlässige, sterile Folie gespannt, der untere Teil der Malzagar Petrischale mit dem Testpilz verkehrt auf die bespannte Trichoderma Petrischale gelegt und verklebt. Die von Trichoderma gebildeten gasförmigen Hemmstoffe diffundierten durch die Folie und hemmten den Testpilz Lentinus lepideus. Der nach 12 Tagen erreichte Koloniedurchmesser von Lentinus lepideus gibt die Hemmwirkung des untersuchten Trichoderma Stammes an.

| | Hemmpilze | Durchmesser der Testpilzkolonie |
|---|---|---|
| Ergebnisse: | Trichoderma harzianum LC F34 | 9 cm |
| | Trichoderma harzianum LC 1 | 5 cm |
| | Trichoderma harzianum LC 2 | 0 cm |
| | Trichoderma harzianum LC 3 | 4,5 cm |

### b) Laboruntersuchung auf antagonistische Hemmwirkung auf verschiedene Schadpilze:

2 %iger Malzagar wurde in einer Petrischale am Rand an 2 gegenüberliegenden Punkten mit je 0,5 cm großen Kolonieplättchen des Hemmpilzes bzw. des Testpilzes beimpft und 2 Wochen bebrütet. Die beiden Kolonien wuchsen aufeinander zu. Erfolgte keine antagonistische Wirkung, so kam das Wachstum der beiden Kolonien bei Berührung zum Stillstand (a). Bei schwacher Hemmwirkung wurde das Mycel des Testpilzes von Trichoderma Überwachsen, sein Wachstum aber nicht abgebrochen (b). Bei starker Hemmwirkung wurde das Mycel des Testpilzes von Trichoderma überwachsen, das Mycel kollabierte und an der Unterseite erfolgte häufig dunkle Pigmentierung (c). Als Vergleichspilz wurde ein Stamm der Art Trichoderma harzianum, die als antagonistisch gilt, herangezogen.

| | T.h. LC F34 | T.sp. LC 1 | T.sp. LC 2 | T.sp. LC 3 |
|---|---|---|---|---|
| Lentinus lepideus | b | c | c | c |
| Coniophora puteana | a | c | c | c |
| Serpula lacrimans | a | c | c | c |
| Armillaria mellea | b | c | c | c |
| Heterobasidion annosum | a | c | c | c |
| Ophiostoma ulmi | b | c | c | c |
| Libertella blepharis | a | b | c | b |
| Botrytis cinerea | a | b | c | b |
| Fusarium oxysporum | b | c | c | c |
| Phytophthora cactorum | b | c | c | c |

### Beispiel 2:

Toxizität einer Sporensuspension von Trichoderma sp. LC 1 mit 10⁸-10¹⁰ Sporen/ml: (Durchgeführt vom Laboratory of Pharmacology and Toxicology, Hamburg, BRD)
- Acute oral toxicity study in the rat: no intolerance reaction
- Acute dermal toxicity study in the rat: no intolerance reactions
- Acute skin tolerance test in rabbits: no irritating properties
- Acute eye irritation study in rabbits: no irritating properties
- Skin sensitation test in the guinea pig: no sensitising properties
- Ames-test: no mutagenic effect

### Beispiel 3:

### EN 113: Bestimmung der Grenze der Wirksamkeit von Trichoderma harzianum LC 1 gegenüber holzzerstörenden Basidiomyceten, die auf Agar gezüchtet werden: (in % Hemmwirkung)

Die Testholzklötzchen wurden mit Sporensuspensionen von Trichoderma sp. LC 1 getränkt bebrütet und normgemäß auf die vorkultivierten Testpilze aufgebracht.

| | 10⁴ Sporen/ml | 10⁶Sporen/ml | 10⁸Sporen/ml |
|---|---|---|---|
| Lentinus lepideus | 100 % | 100 % | 100 % |
| Serpulalacrimans | 100 % | 100 % | 100 % |
| Gloeophyllum trabeum | 100 % | 100 % | 100 % |
| Poria placenta | 100 % | 100 % | 100 % |
| Coniophora puteana | 100 % | 100 % | 100 % |

### Beispiel 4:

### Einsatz von Trichoderma harzianum LC 3 zur Bläuebekämpfung:

Von Trichoderma harzianum LC 3 wurde wie oben beschrieben eine Sporensuspension in einer Konzentration von 10⁸ Sporen/ml hergestellt und diese mit Nährmedium versetzt. Kiefernfurniere aus Splintholz von 4x4 cm wurden in die fungizide Lösung getaucht und in sterilen Petrischalen bei 25 °C bebrütet. Nach 2 Wochen war Trichoderma harzianum LC 3 intensiv angewachsen, aber mit dem freien Auge nicht erkennbar, da die Sporen dieses Pilzes weiß und nicht grün, wie die der anderen Trichoderma Stämme sind. Die bewachsenen Furniere wurden mit einer Sporensuspension von Aureobasidium pullulans, einem Bläuepilz, beimpft.

### Ergebnis:

Behandelte Furniere zeigten keine Färbung durch Trichoderma und nur leichte Färbung durch Aureobasidium. Unbehandelte Furniere wiesen deutliche Blaufärbung auf.

### Beispiel 5:

### Behandlung von Holzstämmen mit einer Sporensuspension von Trichoderma harzianum LC 1:

Stämme aus Kiefernholz von 1 m Länge und ca 20 cm Durchmesser wurden erfindungsgemäß mit einer Suspension bestehend aus 10⁸ Sporen/ml und zugesetzten Nährstoffen getaucht, danach 3 Wochen bei einer Temperatur von 25°C und einer relativen Luftfeuchte von 90 % bebrütet. 5 Stämme wurden bei Raumklima gelagert und weitere 5 Stämme wurden im Garten gemeinsam mit 5 unbehandelten Stämmen von Frühjahr bis Herbst 8 Monate am Boden liegend der Witterung exponiert. Nach 8 Monaten wurden die Stämme optisch beurteilt, mikroskopisch untersucht und Pilze aus dem Holz mikrobiologisch isoliert und nachgewiesen.

### Ergebnis:

### a) Trichoderma harzianum LC 1 behandelt, bei Raumklima gelagert:

Leichte Verblauung an der Oberfläche, sonst keine optischen Veränderungen. Mikroskopie: Pilzhyphen in Splint und Kern zu beobachten. Mikrobiologische Isolation: Trichoderma sp. LC 1 in Splint- und Kernholz nachweisbar.

### b) Trichoderma harzianum LC 1 behandelt, im Freien gelagert:

Mittlere Verblauung an der Oberfläche, sonst keine optischen Veränderungen. Mikroskopie: Pilzhyphen in Splint und Kern zu beobachten. Mikrobiologische Isolation: Trichoderma harzianum LC 1 in Splint- und Kernholz nachweisbar, wenige andere Schimmelpilze.

### c) Unbehandelt, im Freien gelagert:

Starke Verblauung an der Oberfläche, bräunliche Verfärbungen an der Stirnfläche, im Inneren Anzeichen beginnender Braunfäule. Mikroskopie: Verschiedene Pilzhyphen in Splint und Kern zu beobachten, ebenso Schnallenmycelien von Basidiomyceten. Mikrobiologische Isolation: Besonders im Bereich der Stirnseite zahlreiche Schimmelpilze, darunter Bläuepilze. 5 verschiedene Arten von holzzerstörenden Basidiomyceten, darunter mittels Bavendam Test 3 Braunfäulepilze identifiziert.

### Beispiel 6:

Behandlung von Apfelbäumen der Sorte McIntosh mit einem Fungizid, welches Trichoderma harzianum LC 1 und LC 2 enthielt.

Je 500 ml Sporensuspension des Stammes LC 1 und LC 2 wurden zu 1 l Suspension mit einer Konzentration von 10⁸ Sporen/ml vereinigt. Darin wurden sterile Birkenholzstäbe, die zuvor mit Malzbouillon als Nährmedium getränkt wurden, und einen Durchmesser von 5 mm aufwiesen, getaucht. Diese wurden anschließend bei 25°C und 90 % RH 2 Wochen bebrütet.

Zur Bekämpfung des, als Schädling identifizierten Pilzes, Libertella blepharis, wurde die Oberfläche von Ästen an 5 Stellen, wo elliptische Verfärbungszonen - das typische Erscheinungsbild dieser Pilzerkrankung - an der Borke auftraten, mit Alkohol sterilisiert. Danach wurden um die Verfärbungsränder radial zum Stamm 6 Löcher mit 5 mm Durchmesser gebohrt. Die bewachsenen Holzstäbe wurden vor der Applikation in 1,5 %igen Malzagar getaucht und in die Bohrungen inseriert. Die Oberfläche der Bohrung wurde mit Silikonmasse verstrichen. Die 5 behandelten Äste wurden mit 5 nicht behandelten Schadstellen verglichen. Der Beobachtungszeitraum betrug eine Vegatationsperiode.

### Ergebnis:

### Fortschreiten der Verfärbungsränder pro Vegetationsperiode:

| | |
|---|---|
| Unbehandelte Äste | 6,4 ± 1,5 cm |
| Behandelte Äste | 1,9 ± 0,4 cm |

Damit konnte das Fortschreiten der Krankheit in der ersten untersuchten Vegetationsperiode um 70 % reduziert werden.

### Beispiel 7:

Bekämpfung des Wurzelschwammes Heterobasidion annosum im Wurzelraum mit einem Fungizid, welches Trichoderma harzianum LC 1 und LC 2 enthielt.

Der Basidiomycet Heterobasidion annosum wurde auf Petrischalen mit 15 ml Malzagar kultiviert. Nach 3 Wochen wurden die Kolonien von 40 Petrischalen gemeinsam mit dem Agar im Sterilmixer zerkleinert und mit 10 Liter Leitungswasser verdünnt. In 10 Gefäßen wurden je 30 l Erde mit 1 Liter Mycelsuspension beimpft. Die Erde von 5 Gefäßen wurde weiters mit je 1 Liter Sporensuspension von Trichoderma harzianum LC 1 und LC 2 in einer Konzentration von 10⁸ Sporen/ml beimpft, während 5 Gefäße unbeimpft blieben. In jedes Gefäß wurde eine Fichte mit etwa 50 cm Höhe nach guter Durchmischung des Wurzelballens mit der Erde gesetzt. Nach 8 Monaten wurden die Bäumchen aus der Erde genommen und die Wurzeln mikroskopisch und durch Kultivation auf Manifestierung von Heterobasidion annosum an den Wurzeln untersucht.

### Ergebnis:

### Unbehandelte Erde:

An den Wurzeln aller Bäumchen war Heterobasidion nachzuweisen.

### Mit Fungizid behandelte Erde:

Die Wurzeln von 3 Bäumchen waren frei von Heterobasidion, bei den übrigen war bei mikrobiologischen Isolationsversuchen die Häufigkeit des Auftretens um 60 % reduziert.

### Beispiel 8:

Behandlung von Tomatenpflanzen mit einem Fungizid, welches Trichoderma harzianum LC 1 enthält, gegen den Stammfäuleerreger Botrytis cinerea.

Ein Fungizid, erfindungsgemäß eine Sporensuspension von Trichoderma harzianum LC 1 in einer Konzentration von 10⁸ Sporen/ml enthaltend, wurde in einem Glashaus mit kontrollierten Klimabedingungen auf Tomatenpflanzen gesprüht. Es wurden 50 behandelte Pflanzen mit 50 nicht behandelten Pflanzen verglichen.

### Ergebnis:

Unter den nicht behandelten Pflanzen wiesen 8 Pflanzen Stammfäule durch Botrytis cinerea auf. Die Infektionsrate unter den mit dem erfindungsgemäßen Fungizid behandelten Pflanzen war deutlich geringer und betrug 2 infizierte Pflanzen.

## Patentansprüche

1. Pilz Trichoderma harzianum LC 1.

2. Pilz Trichoderma harzianum LC 2.

3. Pilz Trichoderma harzianum LC 3

4. Fungizid, enthaltend eine fungizide Menge eines der oder einer Kombination der Pilze Trichoderma harzianum LC 1, Trichoderma harzianum LC 2., Trichoderma harzianum LC 3.

5. Verfahren zur Bekämpfung von Pilzen, dadurch gekennzeichnet, daß man eine fungizid wirksame Menge der Pilze Trichoderma harzianum LC 1, Trichoderma harzianum LC 2, Trichoderma harzianum LC 3, einzeln oder in Kombination, auf die vor Pilzbefall zu schützenden Hölzer oder Pflanzen vorbeugend oder zur Behandlung eines bereits erfolgten Befalles einwirken läßt.

6. Verfahren nach Anspruch 5, dadurch gekennzeichnet, daß vor Pilzbefall zu schützende Hölzer nach Behandlung mit dem Fungizid bebrütet werden.

7. Verfahren nach einem der Ansprüche 5 oder 6, dadurch gekennzeichnet, daß man Trichoderma harzianum LC 1, LC 2 und/oder LC 3 in einer Konzentration von 10⁴-10¹⁰ koloniebildenden Einheiten je ml Sporensuspension anwendet.

## Claims

1. Fungus Trichoderma harzianum LC 1.

2. Fungus Trichoderma harzianum LC 2.

3. Fungus Trichoderma harzianum LC 3.

4. A fungicide, containing a fungicidal quantity of one or a combination of the fungi Trichoderma harzianum LC 1, Trichoderma harzianum LC 2, Trichoderma harzianum LC 3.

5. A method of controlling fungi, characterized in that a fungicidally effective quantity of the fungi Trichoderma harzianum LC 1, Trichoderma harzianum LC 2, Trichoderma harzianum LC 3, individually or in combination, is allowed to take effect on the timber or plants to be protected from fungal attack preventively or to treat fungal attack that has already taken place.

6. The method according to claim 5, characterized in that the timber to be protected from fungal attack is incubated after the treatment with the fungicide.

7. The method according to one of the claims 5 or 6, characterized in that Trichoderma harzianum LC 1, LC 2 and/or LC 3 is used in a concentration of 10⁴ to 10¹⁰ colony-forming units per ml of spore suspension.

## Revendications

1. Champignon trichoderma harzianum LC 1.

2. Champignon trichoderma harzianum LC 2.

3. Champignon trichoderma harzianum LC 3.

4. Fongicide, comportant une quantité fongicide d'un ou d'une combinaison des champignons trichoderma harzianum LC 1, trichoderma harzianum LC 2, trichoderma harzianum LC 3.

5. Procédé de lutte contre des champignons, caractérisé en ce que l'on fait agir une quantité fongicide efficace des champignons trichoderma harzianum LC 1, trichoderma harzianum LC 2, trichoderma harzianum LC 3, séparément ou en combinaison sur les bois ou les plantes à protéger pour la prévention ou le traitement d'une atteinte fongique existante.

6. Procédé selon la revendication 5, caractérisé en ce que les bois à protéger contre une atteinte fongique sont couvés après le traitement au fongicide.

7. Procédé selon l'une quelconque des revendications 5 ou 6, caractérisé en ce qu'on utilise le trichoderma harzianum LC 1, LC 2 et/ou LC 3 dans une concentration de 10⁴ à 10¹⁰ unités formant des colonies par millilitre de suspension de spores.
